Europäisches Patentamt
European Patent Office
Office européen des brevets

(11) Publication number: **0 038 613**
**B1**

# EUROPEAN PATENT SPECIFICATION

(45) Date of publication of patent specification: **29.08.84**

(51) Int. Cl.[3]: **C 07 C 177/00,** C 07 F 7/18, C 07 D 309/12, A 61 K 31/557

(21) Application number: **81200597.3**

(22) Date of filing: **16.05.80**

(60) Publication number of the earlier application in accordance with Art. 76 EPC: **0 019 475**

## (54) Novel prostaglandins E1 and anti-thrombotic compositions containing them.

(30) Priority: **18.05.79 JP 60293/79**

(43) Date of publication of application: **28.10.81 Bulletin 81/43**

(45) Publication of the grant of the patent: **29.08.84 Bulletin 84/35**

(84) Designated Contracting States: **CH DE FR GB LI**

(56) References cited: **FR-A-2 396 749**

(73) Proprietor: **TEIJIN LIMITED**
**11 Minami Hommachi 1-chome Higashi-ku**
**Osaka-shi Osaka-fu (JP)**

(72) Inventor: **Noyori, Ryoji**
**135-417 Umemori-shinden Nissin-cho**
**Aichi-gun Aichi-ken (JP)**
Inventor: **Suzuki, Masaaki**
**2-66 Yada-cho Higashi-ku**
**Nagoya-shi Aichi-ken (JP)**
Inventor: **Kurozumi, Seizi**
**5-15-6 Tamadaira Hino-shi**
**Tokyo (JP)**

(74) Representative: **Myerscough, Philip Boyd et al**
**J.A.Kemp & Co. 14, South Square Gray's Inn**
**London, WC1R 5EU (GB)**

**EP 0 038 613 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to certain novel prostaglandins $E_1$ and stereoisomers thereof, and to anti-thrombotic compositions containing such prostaglandins as active ingredients.

In our EPC Application No. 80301617.9 (Publication No. 0 019 475 A), from which this Application is divided, we describe and claim a process for producing an adjacently disubstituted ketone of the following formula (5)-a.

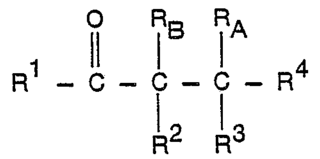

(5)-a

wherein $R^1$, $R^2$, $R^3$ and $R^4$ are identical or different, and represent a hydrogen atom or an organic group having 1 to 20 carbon atoms, or any two of these groups $R^1$, $R^2$, $R^3$ and $R^4$ may be linked to each other to form a ring, $R_A$ represents an organic group having 1 to 20 carbon atoms, and $R_B$ represents a group derived from a protected acetal derivative of an organic carbonyl compound expressed by formula (4)-a below, and when $R^6$ in formula (4)-a represents a hydrogen atom, an organic group having 1 to 20 carbon atoms or the group

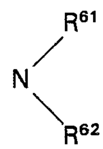

in which $R^{61}$ and $R^{62}$ are identical or different and represent a hydrogen atom or an organic group having 1 to 20 carbon atoms or $R^{61}$ and $R^{62}$ may be linked to each other to form a ring, $R_B$ represents a group resulting from the removal of the group —$OR^7$ from formula (4)-a, and when $R^6$ in formula (4)-a represents —O— ($C_1$—$C_{20}$ organic group) and $R^7$ represents an organic group having 1 to 20 carbon atoms, $R_B$ represents a group resulting from the removal of the group —$OR^7$ or —$R^6$, provided that when two $R^7$ groups are bonded to each other to form a 5- or 6-membered ring, $R_B$ represents a group resulting from the removal of the group —$R^6$ from formula (4)-a, or a group of the following formula:—

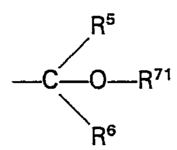

in which $R^5$ and $R^6$ are as defined below and $R^{71}$ represents a $\beta$- or $\gamma$-hydroxyalkyl group, which comprises reacting an $\alpha,\beta$-unsaturated carbonyl compound expressed by the following formula (1):

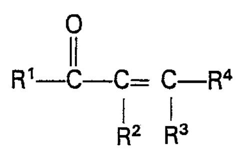

(1)

wherein $R^1$, $R^2$, $R^3$ and $R^4$ are as defined above, with a cuprous salt of the following formula (2):

$$CuX \tag{2}$$

wherein X represents a monovalent anion, and an organolithium compound of the following formula (3):

$$R_ALi \tag{3}$$

wherein $R_A$ is as defined above, in an aprotic inert organic medium in the presence of a trialkylphosphine, the amounts of said cuprous salt and said organolithium compound being substantially equimolar, and thereafter reacting the product with a protected acetal derivative of an organic carbonyl compound of the following formula (4)-a:

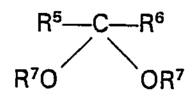

(4)-a

wherein $R^5$ represents a hydrogen atom or an organic group having 1 to 20 carbon atoms, and $R^6$ represents a hydrogen atom, an organic group having 1 to 20 carbon atoms, —O— ($C_1$—$C_{20}$ organic group), or an amino group of the following formula:

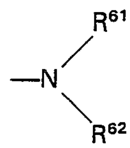

in which $R^{61}$ and $R^{62}$ are as defined above, $R^7$ represents an organic group having 1 to 20 carbon atoms or two $R^7$ groups may be linked to each other to form a 5- or 6-membered ring together with the two oxygen atoms to which they are bonded and the carbon atom to which the two $OR^7$ groups are bonded, and $R^7$ and $R^5$ may be linked to each other to form a 5- or 6-membered ring, in the presence of a Lewis acid.

Among the adjacently disubstituted ketones of formula (5)-a that can be produced by the process of Application No. 80301617.9 are novel prostaglandin analogs, some of which can be used in pharmaceutical compositions as an active ingredient.

The novel prostaglandin analogs provided by the present invention are 7-hydroxyprostaglandins $E_1$, the stereoisomers thereof, or the protected derivatives thereof having the formula (A):

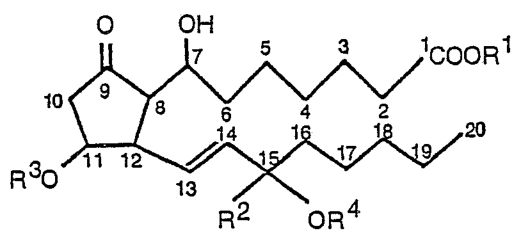

(A)

wherein $R^1$ represents a hydrogen atom, a methyl group or an ethyl group, $R^2$ represents a hydrogen atom or a methyl group, and $R^3$ and $R^4$ are identical or different, and each represents a hydrogen atom, a tetrahydropyranyl group or a t-butyldimethylsilyl group.

Formula (A) embraces compounds of the formulae:

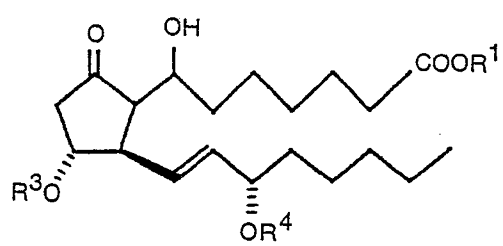

(B)

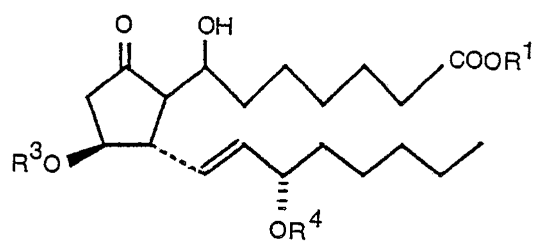

(C)

and

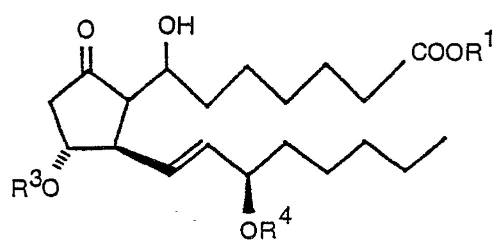

(D)

either singly or as mixtures in arbitrary ratios; $R^1$, $R^3$ and $R^4$ in the three formulae (B), (C) and (D) are as defined with respect to formula (A).

Among the compounds of formula (A) provided by this invention, 7-hydroxyprostaglandin $E_1$ or stereoisomers thereof having the formula (E):

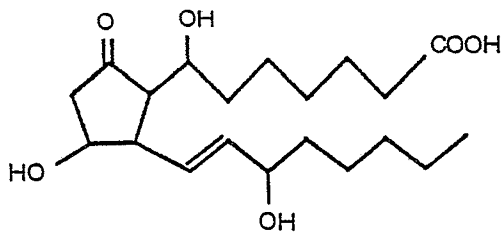

(E)

can be produced by subjecting the 7-hydroxyprostaglandin $E_1$ or its stereoisomer corresponding to formula (A) in which $R^1$, $R^3$, and $R^4$ are protected by groups other than hydrogen and obtained by the process of Application No. 80301617.9 in which the second reaction (with the protected acetal derivative of formula (4)-a) is performed using an acetal derivative of an aldehyde, to a known procedure, for example, hydrolysis under acidic or alkaline conditions, or microbial hydrolysis.

Investigations have led to the discovery that the 7-hydroxyprostaglandin $E_1$ and its stereoisomers of formula (E) have superior anti-thrombotic activity and anti-ulcer activity.

Accordingly, the present invention further provides an anti-thrombotic composition comprising a pharmaceutically effective amount of the 7-hydroxyprostaglandin $E_1$ or a stereoisomer thereof of formula (E) and a pharmaceutically acceptable carrier.

These compounds can be administered to mammals including man when it is desired to inhibit platelet aggregation and to inhibit or prevent thrombus formation. Thus, these compounds are useful for various prophylactic and therapeutic purposes such as the prevention of cardiovascular infarctions and post-operative thrombosis, the promotion of potency of vascular prostheses after surgical operation, and the prevention and treatment of atherosclerosis or arteriosclerosis.

They can also be used to prevent onset of cerebral ischemia in patients suffering from senile diseases, and to prevent or treat myocardial infarction and apoplexy for a long period of time after a seizure.

For these purposes, the compounds of this invention can be administered perorally, intrarectally, or parenterally (e.g., intravenously, subcutaneously, intramuscularly). Preferably, they are administered parenterally.

For peroral administration, the compounds of the invention are formulated into solid or liquid preparations. Solid preparations include tablets, pills, powders and granules. In such solid preparations, at least one active compound is mixed with at least one inert diluent such as calcium carbonate, potato starch, alginic acid or lactose. Other additives such as lubricants (e.g. magnesium stearate) may also be included. Drug formulation is performed in a customary manner.

Liquid preparations for oral administration include emulsions, solutions, suspensions, syrups and elixirs and a general pharmaceutically acceptable inert diluent such as water or liquid paraffin. In addition to the inert diluent, these pharmaceutical preparations contain auxiliary agents such as wetting agents, suspending aids, sweetenings, flavouring agents, perfumes or antiseptics.

The liquid preparations may be encapsulated in absorbable materials such as gelatin.

Solid preparations for intrarectal administration include suppositories containing at least one active compound and prepared by a method known *per se*.

Preparations for parenteral administration are aseptic aqueous or non-aqueous solutions, suspensions or emulsions. Non-aqueous solutions or suspensions contain propylene glycol, polyethylene glycol, vegetable oils such as olive oil, and injectable organic esters such as ethyl oleate. These preparations can also include auxiliary agents such as antiseptics, wetting agents, emulsifiers and dispersing agents. These preparations can be sterilised, for example, by filtration through a bacterium-holding filter, mixing of a bactericide, or by irradiation. Alternatively, aseptic solid preparations are first produced, and immediately prior to use, dissolved in aseptic water or an aseptic solvent for injection.

The dosage of the 7-hydroxyprostaglandin $E_1$ or its stereoisomer, an active compound in accordance with this invention, is about 0.1 $\mu$g to about 100 mg, preferably about 1 $\mu$g to about 10 mg, per day per kilogram of body weight. Of course, the dosage depends upon the condition, body weight and age of a patient and the route of administration.

According to this invention, there is also provided an anti-thrombotic composition containing the 7-hydroxyprostaglandin $E_1$ or its stereoisomer of formula (E) as an inclusion compound of cyclodextrin. By converting the active compound into an inclusion compound of cyclodextrin, the stability of the 7-hydroxyprostaglandin or its stereoisomer increases to make it suitable for oral administration.

The inclusion compound can be prepared by dissolving cyclodextrin in water and/or a water-miscible organic solvent, and adding the solution to a solution of the 7-hydroxyprostaglandin compound in a water-miscible organic solvent. The mixture may be warmed when the desired cyclodextrin inclusion compound can be isolated by concentration under reduced pressure; or the mixture may be cooled and the desired product isolated by filtration or decantation. The ratio of the organic solvent to water varies depending upon the solubilities of the starting materials and the product. Preferably, during the production of the cyclodextrin inclusion compound, the temperature should not exceed 70°C. α-, β-, or γ-cyclodextrin or mixtures thereof can be used in the production of the cyclodextrin inclusion compound.

The following Examples serve to illustrate the invention.

Example 1

Synthesis of 2-(1-hydroxy-7-keto-8-oxanonyl)-3-[3-(tetrahydropyran-2-yloxy)-1-trans-octenyl]-4-(tetrahydropyran-2-yloxy)cyclopentanone (No.1).

Cuprous iodide (325 mg; 1.7 mmoles) was weighed into a 50 ml flask, and the inside atmosphere of the flask was purged with argon. Anhydrous ether (10 ml) was added, and then 687 mg (3.4 mmoles; 0.85 ml) of tri-n-butylphosphine was added at room temperature. The mixture was stirred for 10 minutes. It was then cooled to −78°C, and a solution of 1.7 mmoles of 1-lithio-3-(tetrahydropyran-2-yloxy)-1-trans-octene in a mixture of 10 ml of diethyl ether and 2 ml of pentane was added. The mixture was stirred at −78°C for 30 minutes. A solution of 273 mg (1.5 mmoles) of 4-(tetrahydropyran-2-yloxy)2-cyclopentenone in 5 ml of anhydrous ether was slowly added dropwise. The mixture was stirred for 10 minutes at −78°C, and then 194 mg (1.7 mmoles) of a boron trifluoride-ether complex was added. The mixture was stirred at −78°C for 10 minutes, at −55°C for 20 minutes, and then at −40°C for 10 minutes. Then, 5 ml of anhydrous tetrahydrofuran was added and a solution of 316 mg (2 mmoles) of methyl 7-ketoheptanoate in 10 ml of anhydrous tetrahydrofuran was added. The temperature of the mixture was gradually raised to room temperature. The product was hydrolysed with a saturated aqueous solution of ammonium chloride and extracted with ether. The organic layer was dried over anhydrous sodium sulphate, and concentrated under reduced pressure by an evaporator. The crude product was subjected to silica gel column chromatography (15 g of ammonia-treated silica gel; benzene/ethyl acetate = 3/1 as an eluent) to afford 423 mg (yield 53%) of the captioned compound.

The properties of the product were as follows:

Rf (benzene/ethyl acetate = 1/1 as a developing solvent): 0.43

IR (liquid film):

3440 $cm^{-1}$(OH), 1740 $cm^{-1}$(C=O).

NMR($CCl_4$) δ:

0.8—1.1 (m, 3H, $CH_3$), 1.1—2.8 (m, 35H, $CH_2$, CH and OH), 3.2—4.2 (m, 7H, $OCH_2CH_2$, CHOTHP and CHOH), 3.62 (s, 3H, $OCH_3$) 4.4—4.8 (m, 2H, OCHO), 5.4—5.8 (m, 2H, CH=CH).

Treatment of this product in a tetrahydrofuran solvent in a customary manner afforded 2-(1-hydroxy-7-keto-8-oxanonyl)-3-(3-hydroxy-1-trans-octenyl)-4-hydroxy-cyclopentanone (dl-7-OHPG$E_1$ methyl ester) (No. 2).

Example 2

Synthesis of dl 2-(1-hydroxy-7-keto-8-oxanoyl)-3-[3-(t-butyldimethylsilyloxy)-1-trans-octenyl]-4-(t-butyldimethyl-silyloxy)-cyclopentanone (Nos. 3 and 4), and dl 2-(1-hydroxy-7-keto-8-oxanonyl)-3-(3-hydroxy-1-trans-octenyl)-4-hydroxycyclopentanone (Nos. 5, 6, 7 and 8):—

Cuprous chloride (229 ml; 1.2 mmoles) was weighed into a 50 ml flask, and the inside atmosphere of the flask was purged with argon. Anhydrous ester (10 ml) was added, and then 0.60 ml (2.4 mmoles) of tri-n-butyl-phosphine was added at room temperature, followed by stirring for 10 minutes. The mixture was then cooled to −78°C, and a solution of 1.2 mmoles of dl 1-lithio-3-(t-butyldimethylsilyloxy)-trans-octene in a mixture of anhydrous ether and anhydrous hexane was added. The mixture was stirred at −78°C for 30 minutes, and a solution of 212 mg (1.0 mmole) of dl 4-(t-butyldimethylsilyloxy)-2-cylopentenone in 5 ml of anhydrous ether was added dropwise. The mixture was stirred at −78°C for 1 hour. Then, 0.15 ml of a boron trifluoride/ether complex was added, and the mixture was stirred at −78°C for 10 minutes, and then at −40°C for 20 minutes. Then, 5 ml of anhydrous tetrahydrofuran was added, and a solution of 221 mg (1.4 mmoles) of methyl 7-ketoheptanoate in 5 ml of anhydrous tetrahydrofuran was added. The mixture was stirred at −40°C for 1.5 hours. The temperature of the mixture was gradually raised to room temperature, and the product was hydrolysed with a saturated aqueous solution of ammonium chloride and extracted with ether. The organic layer was dried over anhydrous sodium sulphate, and concentrated under reduced pressure. The crude product was subjected to silica gel column chromatography. From the eluates resulting from elution with hexane/ethyl acetate (=9/1), two stereoisomers, dl 2-(1-hydroxy-7-keto-8-oxanonyl)-3-[3-(t-butyldimethylsilyloxy)-1-trans-octenyl]-4-(t-butyldimethylsilyloxy)cyclopentanone, were obtained in an amount of 59 mg (TLC: Rf=0.39; developing solvent hexane/ethyl acetate = 4/1) and 35 mg (TLC: Rf = 0.35; developing solvent hexane/ethyl acetate = 4/1), respectively.

The properties of these products were as follows:

Stereoisomer (No. 3) having an Rf value of 0.39

IR (liquid film):

3470, 2960, 2945, 2870, 1741, 1461, 1436, 1404, 1388, 1360, 1251, 1196, 1170, 1104, 1002, 970, 938, 834, 772, 665 $cm^{-1}$.

NMR($CCl_4$) $\delta$:

0.85 (s,18H), 3,62 (s, 3H), 3.85—4.3 (m, 2H), 5.46—5.70 (m, 2H).

Stereoisomer (No. 4) having an Rf value of 0.35

IR (liquid film):

3470, 2960, 2940, 2870, 1742, 1464, 1438, 1410, 1378, 1360, 1258, 1100, 1006, 970, 938, 876, 836, 810, 778, 668 $cm^{-1}$.

NMR($CCl_4$) $\delta$:

0.88 (s, 18H), 3.60 (s, 3H), 3.4 (m, 1H) 3.7—4.3 (m, 2H), 5.4—5.7 (m, 2H).

These two compounds were each subjected to desilylation reaction in a customary manner (THF-water-AcOH = 1:1:3; reacted at room temperature for 5 days). The solvent was removed by distillation under reduced pressure, and the product was separated by silica gel column chromatography (benzene/ethyl acetate = 1/1) to afford dl 2-(1-hydroxy-7-keto-8-oxanonyl)-3-(3-hydroxy-1-trans-octenyl)-4-hydroxycylopentane.

The properties of the products were as follows:

[Desilylation product of compound (No. 3)]

Compound having a larger Rf value (dl 15-epi-7-OHPG$E_1$ methyl ester) (No. 5); yield 20%

NMR($CDCl_3$) $\delta$:

0.89 (m, 3H), 1.1—1.8 (m, 16H), 2.0—2.95 (m, 9H), 3.66 (s, 3H), 3.6—3.85 (m, 1H), 3.9—4.25 (m, 2H), 5.4—5.9 (m, 2H).

Compound having a smaller Rf value (dl 7-OHPG$E_1$ methyl ester) (No. 6); yield: 28%

NMR($CDCl_3$) $\delta$:

0.89 (m, 3H), 1.0—1.7 (m, 16H), 1.9—2.9 (m, 6H), 3.05 (s, 3H, disappeared upon addition of $D_2O$), 3.66 (s, 3H), 3.5—3.8 (m, 1H), 3.9—4.2 (m, 2H), 5.45—5.7 (m, 2H).

[Desilylation product of compound No. 4]

Compound having a larger Rf value (dl 15-epi-7-OHPG$E_1$ methyl ester) (No. 7); yield 18%

NMR($CDCl_3$) $\delta$:

0.89 (m, 3H), 1.0—1.8 (m, 16H), 1.95—3.6 (m, 9H), 3.67 (s, 3H), 3.3—4.3 (m, 3H), 5.4—5.85 (m, 2H).

Compound having a smaller Rf value (dl 7-OHPG$E_1$ methyl ester) (No. 8); yield: 14%

NMR($CDCl_3$) $\delta$:

0.89 (m, 3H), 1.1—1.8 (m, 16H), 2.0—3.0 (m, 9H), 3.67 (s, 3H), 3.85—4.30 (m, 3H), 5.55—5.75 (m, 2H).

## Example 3

Synthesis of 2-(1-hydroxy-7-keto-8-oxanonyl)-3-[3(S)-(t-butyldimethylsilyloxy)-1-trans-octenyl]-4-(R)-(t-butyldimethylsilyloxy) cyclopentanone (Nos. 9 and 10) and 2-(1-hydroxy-7-keto-8-oxanonyl)-3-[3(S)-hydroxy-1-trans-octenyl]-4(R)-hydroxycyclopentanone (Nos. 11 and 12):—

Cuprous iodide (343 mg; 1.8 mmoles) was weighed into a 50 ml flask, and the inside atmosphere of flask was purged with argon. Anhydrous ether (15 ml) was added, and 0.9 ml (3.6 mmoles) of tri-n-butylphosphine was added at room temperature, followed by stirring for 10 minutes. The mixture was cooled to −78°C, and a solution of 1.8 mmoles of 1-lithio-3(S)-(t-butyldimethylsilyloxy)-trans-octene in a mixture of anhydrous ether and anhydrous hexane was added. The mixture was stirred at −78°C for 30 minutes, and then, a solution of 318 mg (1.5 mmoles) of 4(R)-(t-butyldimethylsilyloxy)-2-cyclopentenone in 7 ml of anhydrous ether was added dropwise. The mixture was stirred at −78°C for 1 hour. To the mixture was added 0.23 ml of a boron trifluoride-ether complex, and the mixture was stirred at −78°C and then at −40°C for 20 minutes. Anhydrous tetrahydrofuran (5 ml) was added, and a solution of 332 mg (2.1 mmoles) of methyl 7-keto-heptanoate in 7 ml of anhydrous tetrahydrofuran was added. The mixture was stirred at −40°C for 1.5 hours. The temperature of the mixture was gradually raised to room temperature, and the product was hydrolysed with a saturated aqueous solution of ammonium chloride, and extracted with ether. The organic layer was dried over anhydrous sodium sulphate, and concentrated under reduced pressure. The crude product was subjected to silica gel column chromatography. From the eluates resulting from elution with hexane/ethyl acetate (=9/1), two stereoisomers, 2-(1-hydroxy-7-keto-8-oxanonyl)-3-[3(S)-(t-butyldimethylsilyloxy)cyclopentanone, were obtained in an amount of 79 mg (TLC: Rf=0.39; developing solvent hexane/ethyl acetate=4/1; compound No. 9), and 75 mg (TLC: Rf=0.35; developing solvent hexane/ethyl acetate = 4/1; compound No. 10), respectively.

The Rf value and spectral data of compound No. 9 corresponded with those of compound No. 3 obtained in Example 2, and the Rf value and spectral data of compound No. 10, with those compound No. 4 obtained in Example 2.

Then, the compounds Nos. 9 and 10 were each subjected to desilylation reaction to afford 2-(1-hydroxy-7-keto-8-oxanonyl)-3-[(S)-hydroxy-1-trans-octenyl]-4(R)-hydroxycyclopentanone.

The Rf value and spectral data of 7-OHPGE$_1$ methyl ester (No. 11) obtained by desilylation of compound No. 9 corresponded with those of compound No. 6 obtained in Example 2, and the Rf value and spectral data of an epimer of compound No. 11 (No. 12) obtained by desilylation of compound No. 10, with those of compound No. 8 obtained in Example 2.

Example 4

By the method of Y. H. Lee *et al.*, [Arch. Int. Pharmacodyn. Ther., *192*, 370 (1971), the inhibiting action of the compound No. 2 in Example 1 and compound No. 12 in Example 3 on the formation of ulcer in rats induced by indomethacin, a typical non-steroid anti-inflammatory agent, was evaluated. As shown in Table 1 below, this compound inhibited ulcer formation.

Male rats (Sprague Dawley species; body weight 240—270 g) were used in the experiment. These rats had been caused to fast for 24 hours in separate cages before the initiation of the experiment. Indomethacin was orally administered to these rats, and 6 hours later, they were killed. Formation of ulcer in the fundus part was examined by measuring the area of the ulcer-forming part under a microscope. The compounds Nos. 2 and 12 were each subcutaneously administered at the end of 30 minutes and 2 hours, respectively, after the administration of indomethacin. The ulcer formation inhibiting rate of these compounds against a predetermined dose of indomethacin was determined.

The indomethacin was administered as a 0.5 ml aqueous suspension (containing two drops of a Tween-type non-ionic surfactant per 14 ml of water). The compounds Nos. 2 and 12 were given in the form of a solution in a phosphate buffer (pH 7.4).

Table 1 shows the inhibition, by compound Nos. 2 and 12, of ulcer formation induced by indomethacin (20 mg/kg, peroral).

TABLE 1

| Compound | Dose (mg/kg, S.C.) | Number of rats | Ulcer formation (ulcer coefficient) Average value ± S.E.M. | Inhibiting rate (%) |
|---|---|---|---|---|
| None (control) | Zero | 12 | 20.3 ± 4.4 | 0 |
| (2) | 0.5 × 2 | 6 | 12.1 ± 4.1 | 40.4 |
| (12) | 0.5 × 2 | 5 | 15.8 ± 5.1 | 22.2 |

S.E.M. shows a standard error of the mean value.

Example 5

Activity of inhibiting platelet aggregation *in vitro*:—

The platelet aggregation inhibiting activities of test compounds including 7-hydroxy PGE$_1$ of this invention were tested using rabbit PRP. The results are shown in terms of their concentrations at 50% inhibition ($IC_{50}$) of platelet aggregation. As an aggregating agent, ADP- disodium salt having a final concentration of 5 $\mu$m was used.

Preparation of PRP, test compounds, and aggregating agent

(1) Preparation of platelet-enriched plasma (PRP)

Citrated blood (consisting of 1 part by volume of 38% sodium citrate and 9 parts by volume of the blood) was prepared by taking blood from the ear veins of male rabbits having a body weight of 2.0 to 3.0 kg (white ordinary species). The citrated blood was centrifuged at 100G for 10 minutes at room temperature, and the supernatant (PRP) was separated.

The PRP obtained was stored at room temperature, and used as early as possible. PRP stored for more than 4 hours after preparation was not used.

(2) Preparation of test drugs

Each test drug was dissolved in ethyl alcohol to a concentration of 10 mg/ml or 1 mg/ml, and diluted with physiological saline to adjust its concentration.

(3) Preparation of the aggregating agent

ADP solution

ADP disodium salt (a product of Kyowa Hakko Kabushiki Kaisha) was dissolved in 0.1M tris-HCl

buffer (pH 7.8) to prepare a solution having a concentration of 50 μm (final concentration 5 μm).

Testing method for inhibition of platelet aggregation

(1) Degree of platelet aggregation in a blank

Physiological salt (25 μl) and 25μl of the aggregating agent solution were added to 200 μl of PRP which had been warmed in a cuvette at 37°C of an aggregometer to aggregate platelet. The aggregation curve was recorded for 3 minutes by an aggregometer (a product of Rika Denki Kabushiki Kaisha). The maximum degree of aggregation in this platelet aggregation was defined as the maximum degree of aggregation of the blank.

(2) Test for inhibition of platelet aggregation

The solution of test drug (25 μl) was added to 200 of PRP, and the mixture was pre-incubated for 2 minutes in a cuvette of an aggregometer. Then, 25 μl of the ADP disodium solution (50 μM) was added, and the aggregation curve was recorded for 3 minutes. The maximum degree of platelet aggregation within this period was measured, and the inhibiting rate was calculated from the following equation. The minimum concentration (expressed as final concentration) of the drug whose inhibiting rate exceeded 50% as shown as an $IC_{50}$ value.

$$\text{Inhibiting rate (\%)} = \frac{\text{Maximum degree of aggregation in the drug-added system}}{\text{Maximum degree of aggregation of the blank}} \times 100$$

For details of the *in vitro* platelet aggregation inhibiting test, see H. M. Davis, J. Phyllis, K. Paul and W. Terry: Thromb. Res. *11*, 217—226 (1977) and R. E. Anderson and J. G. Foulks: Thromb. Haemos. *36*, 343—359 (1976).

The results are shown in Table 2

TABLE 2

| Run No. | 7-Hydroxy $PGE_1$ methyl ester or its stereoisomer | $IC_{50}$ (μg/ml) |
|---|---|---|
| 1 | No. 5 | > 10 |
| 2 | No. 6 | 24.5 |
| 3 | No. 7 | > 10 |
| 4 | No. 8 | > 10 |
| 5 | No. 11 | 26.3 |
| 6 | No. 12 | 41.5 |

Example 6

Formulation of tablets:—

Tablets were prepared each of which had the following composition.

| | |
|---|---|
| Active ingredient | 200 mg |
| Lactose | 280 mg |
| Potato starch | 80 mg |
| Polyvinyl pyrrolidone | 11 mg |
| Magnesium stearate | 5 mg |
| | 576 mg |

The active ingredient, lactose and potato starch were mixed, and the mixture uniformly wetted with a 90% ethanol solution of polyvinyl pyrrolidone. The wet mixture was passed through a 2.0 mm-mesh sieve, and dried at 45°C, and again passed through a 1.5 mm-mesh sieve. The granules obtained were mixed with magnesium stearate, and the mixture compressed into tablets.

As representative active ingredients, compounds Nos. 11 and 12 were used respectively.

Example 7

Formulation of capsules:—

Hard gelatin capsules were produced each of which had the following recipe.

| | |
|---|---|
| Active ingredient | 200 mg |
| Microcrystalline cellulose | 195 mg |
| Amorphous silica | 5 mg |
| | 400 mg |

The active ingredient in a finely divided form, the microcrystalline cellulose and the unpressed amorphous silica were fully mixed, and packed into hard gelatin capsules.

As representative active ingredients, compounds Nos. 11 and 12 were used respectively. No substantial trouble occurred during manufacture.

Example 8

Ampoules were prepared each of which had a capacity of 5 ml and contained the following ingredients in the proportions indicated.

| | |
|---|---|
| Active ingredient | 200 mg |
| Polyethylene glycol 600 | 200 mg |
| Distilled water to make | 5 ml |

The active ingredient and polyethylene glycol were dissolved in water in an atmosphere of nitrogen. The solution was boiled, and cooled under a nitrogen atmosphere, and distilled. Pre-treated water was added to the solution to the desired capacity, and filtered aseptically. The above manufacturing operation was performed under scattered light. Filling of the solution was carried out in a stream of nitrogen, and sterilisation was performed by heating at 121°C for 20 minutes.

As representative active ingredients, compounds Nos. 11 and 12 were used.

Example 9

Production of 7-hydroxy $PGE_1$-cyclodextrin inclusion compound:—

Ten milligrams of 7-Hydroxy $PGE_1$ was dissolved in 0.1 ml of ethanol, and the solution was added to a solution of 15 mg of $\beta$-cyclodextrin in 0.2 ml of water. The mixture was stirred at room temperature for 10 minutes. The resulting mixture was concentrated under reduced pressure to afford 13 mg of a 7-hydroxy $PGE_1$-$\beta$-cyclodextrin inclusion compound. The product contained 6% of 7-hydroxy $PGE_1$.

**Claims**

1. 7-Hydroxyprostaglandin $E_1$, or a stereoisomer thereof, or a protected derivative thereof, having the formula:

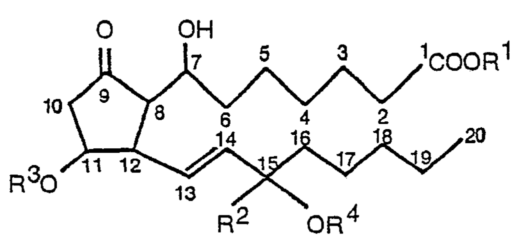

wherein $R^1$ represents a hydrogen atom, a methyl group or an ethyl group, $R^2$ represents a hydrogen atom or a methyl group, and $R^3$ and $R^4$ are identical or different, and each represents a hydrogen atom, a tetrahydropyranyl group or a t-butyldimethylsilyl group.

2. A compound according to claim 1 having the formula:

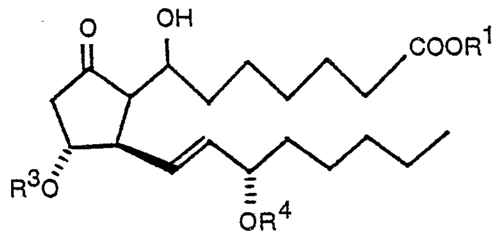

wherein $R^1$, $R^3$ and $R^4$ are as defined in claim 1.

3. A compound according to claim 1 having the formula:

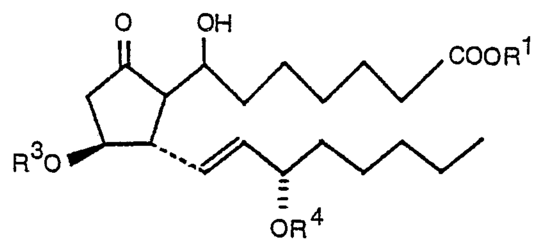

wherein $R^1$, $R^3$ and $R^4$ are as defined in claim 1.

4. A compound according to claim 1 having the formula:

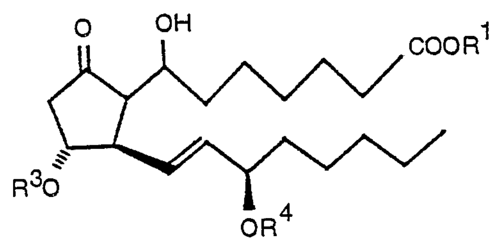

wherein $R^1$, $R^3$ and $R^4$ are as defined in claim 1.

5. An anti-thrombotic composition comprising a pharmaceutically effective amount of 7-hydroxyprostaglandin $E_1$ or a stereoisomer thereof of the formula:

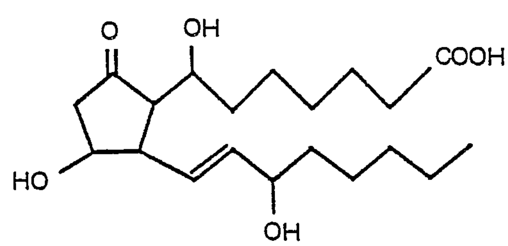

and a pharmaceutically acceptable carrier.

6. An anti-thrombotic composition according to claim 5 containing 7-hydroxyprostaglandin $E_1$ or its stereoisomer as an inclusion compound of cyclodextrin.

**Patentansprüche**

1. 7-Hydroxyprostaglandin $E_1$ oder ein Stereoisomers davon oder ein geschütztes Derivat davon mit der allgemeinen Formel

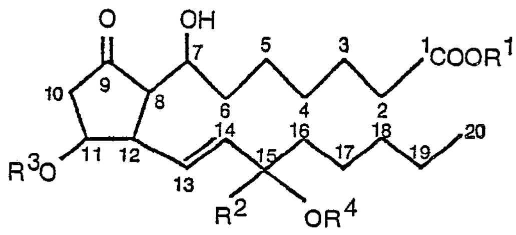

in der $R^1$ ein Wasserstoffatom, eine Methylgruppe oder eine Ethylgruppe bedeutet, $R^2$ ein Wasserstoffatom oder eine Methylgruppe ist und $R^3$ und $R^4$ gleich oder verschieden sind und jeweils ein Wasserstoffatom, eine Tetrahydropyranylgruppe oder eine tert.-Butyldimethylsilylgruppe bedeuten.

2. Verbindung nach Anspruch 1, entsprechend der Formel

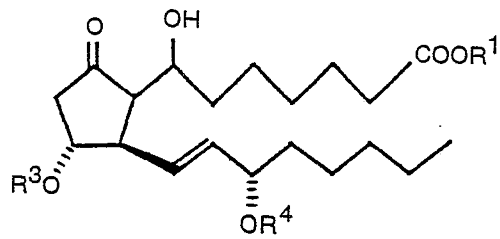

in der $R^1$, $R^3$ und $R^4$ die in Anspruch 1 angegebene Bedeutung haben.

3. Verbindung nach Anspruch 1, entsprechend der Formel

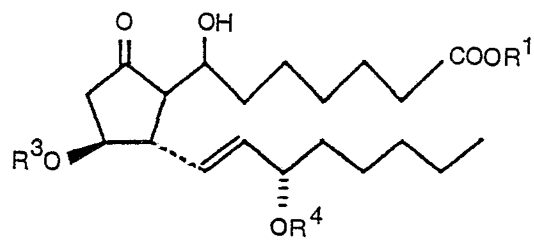

in der $R^1$, $R^3$, und $R^4$ die in Anspruch 1 angegebene Bedeutung haben.

4. Verbindung nach Anspruch 1, entsprechend der Formel

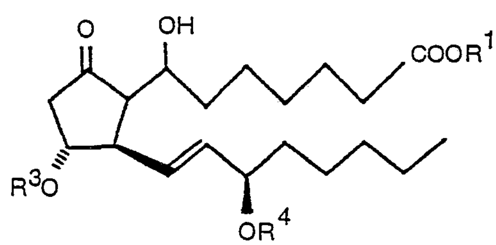

in der $R^1$, $R^3$ und $R^4$ die in Anspruch 1 angegebene Bedeutung haben.

5. Antithrombotisches Mittel, umfassend eine pharmazeutisch wirksame Menge 7-Hydroxyprostaglandin $E_1$ oder eines Stereoisomeren davon, entsprechend der Formel

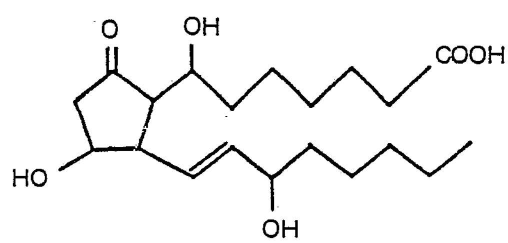

und einen pharmazeutisch annehmbaren Träger.

6. Antithrombotisches Mittel nach Anspruch 5, enthaltend 7-Hydroxyprostaglandin $E_1$ oder dessen Stereoisomer als Einschlußverbindung von Cyclodextrin.

**Revendications**

1. 7-Hydroxyprostaglandine $E_1$, ou un stéréo-isomère de celle-ci, ou un dérivé protégé de celle-ci ayant la formule:

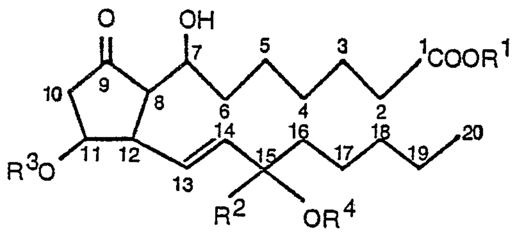

dans laquelle $R^1$ représente un atome d'hydrogène, un groupe méthyle ou un groupe éthyle; $R^2$ représente un atome d'hydrogène ou un groupe méthyle, et $R^3$ et $R^4$ sont identiques ou différents et chacun représente un atome d'hydrogène, un groupe tétrahydropyranyle ou un groupe t-butyldiméthylsilyle.

2. Composé selon la revendication 1, ayant la formule:

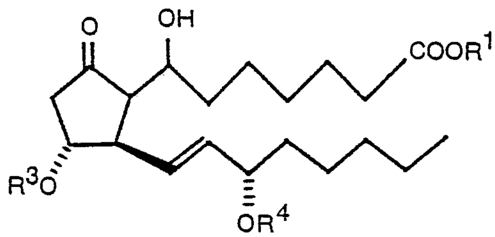

dans laquelle $R^1$, $R^3$ et $R^4$ ont les définitions données dans la revendication 1.

3. Composé selon la revendication 1 ayant la formule:

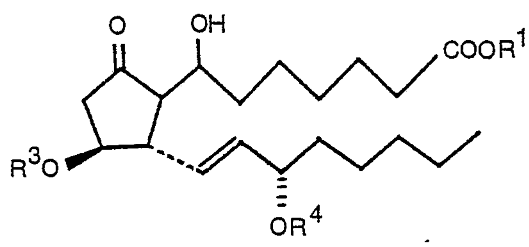

dans laquelle $R^1$, $R^3$ et $R^4$ ont les définitions données dans la revendication 1.

4. Composé selon la revendication 1 ayant la formule:

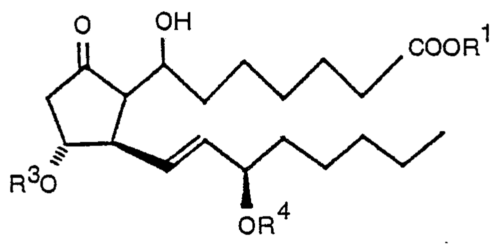

dans laquelle $R^1$, $R^3$ et $R^4$ ont les définitions données dans la revendication 1.

5. Composition antithrombotique comprenant une quantité efficace pharmaceutiquement de 7-hydroxyprostaglandine $E_1$, ou un stéréoisomère de celle-ci, ayant la formule:

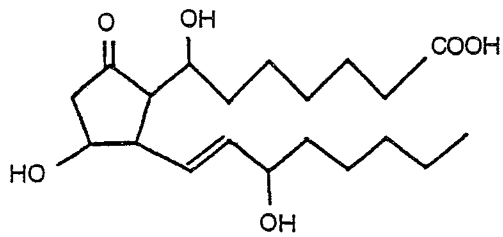

et un véhicule pharmaceutiquement acceptable.

6. Composition antithrombotique selon la revendication 5, contenant la 7-hydroxyprostaglandine $E_1$ ou son stéréoisomère comme composé d'introduction de cyclodextrine.